# EUROPEAN PATENT APPLICATION

(11) **EP 3 698 648 A1**
(43) Date of publication of application: **26.08.2020**
(21) Application number: 19158816.9
(22) Date of filing: 22.02.2019
(51) Int. Cl.: A23L 29/30, C07H 3/02, A23K 20/163, A23L 33/125

(54) **ALLULOSE PET FOOD**

(71) Applicant: Pfeifer & Langen GmbH & Co. KG, 50858 Köln (DE)
(72) Inventor: KAUFMANN, Birgit, 50933 Köln (DE); DEGENHARDT, Andreas Georg, 50374 Erftstadt (DE); KOCH, Timo Johannes, 50189 Elsdorf (DE)
(74) Representative: Kutzenberger Wolff & Partner

(57) **Abstract**

The invention relates to a pet food composition comprising D-allulose. It has been found that D-allulose is capable of rendering pet food with prebiotic properties, stimulating the production of organic acids in the colon by indigenous bacteria, selectively stimulating and/or suppressing the growth or activity of indigenous bacteria in the colon, or improving the balance of the intestinal bacterial flora. The invention also relates to an edible pet food composition comprising D-allulose in combination with another prebiotic, or in combination with another food ingredient for which an EU Health Claim has been acknowledged, or in combination with a probiotic.

## Description

The invention relates to pet food, more particularly to food for dogs and cats, comprising D-allulose.

Pet food is processed from fish, meat, milk and the like with additional dairy products, vegetables, cereals vitamins and so on. There are many types of pet food such as dry-type food in which less than 10 wt.-% of water is contained, semi-moist-type food in which 15-30 wt.-% of water is contained and wet-type food in which more than 75 wt.-% of liquid, i.e. water or milk is contained. These types of pet food, especially wet-type cat food containing more than 70 wt.-% water or milk, are packed in containers by about 100 g.

US 2014/0271996 discloses beverages comprising rare sugars and sweetness enhancers. In one embodiment the rare sugar is selected from the group consisting of D-allose, D-psicose, L-ribose, T-tagatose, L-glucose, L-fucose, L-arabinose, D-turanose, D-leucrose and combinations thereof.

Nishii et al., J. Vet. Med. Sci. 78(6), 2016, p. 1079-1083 relates to the evaluation of toxicity of D-allulose administered to healthy dogs investigated by blood glucose- and plasma insulin concentration monitoring 2 h after administration of D-allulose.

Nishii et al., J. Vet. Med. Sci. 78(6), 2016, p. 1657-1662 relates to the effects of D-allulose on glucose metabolism after the administration of sugar or food in healthy dogs.

Nishii et al., J. Vet. Med. Sci. 79(11), 2017, p. 1780-1784 relates to the safety and biological effects of a long-term dose of D-allulose in healthy dogs.

WO 2017/029245A1 relates to the use of allulose or of an edible composition comprising allulose as prebiotic.

Due to the sugar content of pet food, cats and dogs tend to obesity, diabetes and related diseases which is unpleasant for the owner and may create undesired health expenditures. Further, feces and urine of cats and dogs may have a foul smell leading to unpleasant room odors. Such diseases and of cats and dogs as well as unpleasant smells are of a great concern for their owners. Thus, there has been a long-felt need for preventing such diseases and reducing the smell emissions.

Thus, it is an object of the present invention to provide pet food having improved characteristics compared to pet food known from prior art.

This object has been achieved by the subject-matter of the patent claims.

A first aspect of the invention relates to a pet food composition containing
- D-allulose at a content of at least 0.025 wt.-%,
- fat at a content within the range of from 0.1 wt.-% to 48 wt.-%,
- protein at a content within the range of from 0.1 to 75 wt.-%, preferably from 5.0 wt.-% to 75 wt.-%, and
- carbohydrate other than D-allulose at a content within the range of from 1.0 wt.-% to 85 wt.-%,
in each case based on the total weight of the pet food composition on dry-matter basis.

It has been surprisingly found that D-allulose has a sweetening capacity that is about 70 wt.-% of that of sucrose but at the same time has no or nearly no physiological caloric value, therefore reducing the risk of diseases such as obesity and diabetes.

Still further, it has been surprisingly found that D-allulose is not fermented by most microorganisms that are conventionally used in the production of fermented foodstuff, beverages and the like. Thus, in the presence of such microorganisms, D-allulose remains inert, i.e. storage stable, which has an overall positive effect on the preservation of the pet food.

Yet further, it has been surprisingly found that D-allulose does not have a pronounced laxative effect.

Furthermore, it has been surprisingly found that the reaction products achieved by heating edible compositions containing D-allulose including but not limited to Maillard products and caramelization products, respectively, which can but do not need to have brownish color, provide a particular taste to the pet food composition that is liked by many pets thus rendering the pet food composition more attractive.

Moreover, it has been surprisingly found that pet food compositions according to the invention not containing significant amounts of digestible mono- or disaccharides other than D-allulose such as glucose, fructose, sucrose and lactose are nonetheless well accepted by various pets due to the presence of D-allulose. Thus, the present invention provides well accepted pet food compositions that compared to conventional pet food compositions have health benefits e.g. with regard to calorie content.

In the context of the present invention, pet food typically refers to products of vegetable or animal origin, which are consumed by animals, according to the invention pets, and contribute to energy and/or nutrient to the animal's diet.

The pet food composition according to the invention contains D-allulose. D-allulose, also referred to as D-psicose, is a ketohexose. D-allulose (CAS no. 551-68-8) in open chain Fischer projection has the following structure:

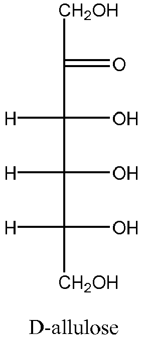

The D-allulose is typically present in form of the monosaccharide, i.e. in open chain form, furanose form or pyranose form, but typically not glycosylated with other carbohydrates.

The pet food composition according to the invention contains fat. The term "fat" typically describes a triacylglycerol mixture which according to the invention may be liquid (oils), semi-solid or solid at room temperature (25 °C). For the purpose of the specification, the term "fat" also encompasses lipoids such as phospholipids, sphingolipids, and steroids.

The pet food composition according to the invention contains protein, i.e. typically a (poly)peptide in which at least 40 amino acid residues are present.

The pet food composition according to the invention contains carbohydrates other than D-allulose. Typical "carbohydrates" include monosaccharides (e.g. glucose, fructose), disaccharides (e.g. sucrose), oligosaccharides, and polysaccharides.

Unless otherwise stated, the content of the ingredients is given in weight percent on dry-matter basis. The term "dry-matter basis" describes the amount of a particular nutrient in percentages as found in the dry weight of the pet food, i.e. when all moisture is removed.

The pet food composition according to the invention is devoted for pets which are not particularly limited. Preferred pets (domestic animals, farm animals) according to the invention include but are not limited to dogs, cats, mice, rats, rabbits, hamster, guinea pigs, birds, fish and the like. Preferred pets are selected from cats, dogs, rabbits, hamsters and guinea pigs. Particularly preferred pets within the present invention are cats and/or dogs.

Preferably, the pet food composition according to the invention has a D-allulose content of at least 0.05 wt.-%, more preferably of at least 0.075 wt.-%, even more preferably of at least 0.1 wt.-%, based on the total weight of the pet food composition on dry-matter basis.

Preferably, the pet food composition according to the invention has a D-allulose content of at most 5.0 wt.-%, more preferably of at most 4.5 wt.-%, even more preferably of at most 4.0 wt.-%, still more preferably of at most 3.5 wt.-%, yet more preferably 3.0 wt.-% and in particular 2.5 wt.-%, based on the total weight of the pet food composition on dry-matter basis.

In a preferred embodiment, the D-allulose content of the pet food composition according to the invention is in a range from 0.025 wt.-% to 5.0 wt.-%, more preferably from 0.025 wt.-% to 4.5 wt.-%, still more preferably from 0.025 wt.-% to 4.0 wt.-%, even more preferably from 0.025 wt.-% to 3.5 wt.-%, yet more preferably from 0.025 wt.-% and 3.0 wt.-% and in particular from 0.025 wt.-% to 2.5 wt.-%, based on the total weight of the pet food composition on dry-matter basis.

In another preferred embodiment, the D-allulose content of the pet food composition according to the invention is in a range from 0.05 wt.-% to 5.0 wt.-%, more preferably from 0.05 wt.-% to 4.5 wt.-%, still more preferably from 0.05 wt.-% to 4.0 wt.-%, even more preferably from 0.05 wt.-% to 3.5 wt.-%, yet more preferably from 0.05 wt.-% and 3.0 wt.-% and in particular from 0.05 wt.-% to 2.5 wt.-%, based on the total weight of the pet food composition on dry-matter basis.

In yet another preferred embodiment, the D-allulose content of the pet food composition according to the invention is in a range from 0.1 wt.-% to 5.0 wt.-%, more preferably from 0.1 wt.-% to 4.5 wt.-%, still more preferably from 0.1 wt.-% to 4.0 wt.-%, even more preferably from 0.1 wt.-% to 3.5 wt.-%, yet more preferably from 0.1 wt.-% to 3.0 wt.-% and in particular from 0.1 wt.-% to 2.5 wt.-%, based on the total weight of the pet food composition on dry-matter basis.

In still another preferred embodiment, the D-allulose content of the pet food composition according to the invention is in a range from 0.025 wt.-% to 5.0 wt.-%, more preferably from 0.05 wt.-% to 4.5 wt.-%, still more preferably from 0.075 wt.-% to 4.0 wt.-%, even more preferably from 0.1 wt.-% to 3.5 wt.-%, based on the total weight of the pet food composition on dry-matter basis.

In another preferred embodiment, the D-allulose content of the pet food composition according to the invention is in a range from 0.125 wt.-% to 3.0 wt.-% and more preferably from 0.15 wt.-% to 2.5 wt.-%, based on the total weight of the pet food composition on dry-matter basis.

In a preferred embodiment, the pet food composition according to the invention contains fat at a content within the range of from 8.0 wt.-% to 30 wt.-%, more preferably from 8.0 wt.-% to 25 wt.-%, even more preferably from 10 wt.-% to 32 wt.-%, still more preferably from 10 wt.-% to 30 wt.-%, yet more preferably from 15 wt.-% to 30 wt.-%, in each case based on the total weight of the pet food composition on dry-matter basis.

Suitable types of fat for the pet food composition include dairy-based fat and/or vegetable-based fat and /or animal-based fat.

Non-limiting examples of dairy-based fats are butter, cheese, milk fat and mixtures thereof.

Non-limiting examples of vegetable-based fats are liquid, solid, and semi-solid hydrogenated or partially hydrogenated vegetable oil such as palm oil, coconut oil, cottonseed oil, soybean oil, peanut oil, flax seed oil, grape seed oil, olive oil, and mixtures thereof.

Non-limiting examples of animal-based fats are tallow, lard, chicken fat, fish oil, and mixtures thereof. Typically, the pet food composition will comprise a plant derived fat source when it is formulated as a vegetarian pet food.

In a preferred embodiment, the pet food composition according to the invention contains protein at a content within the range of from 18 wt.-% to 45 wt.-%, more preferably from 18 wt.-% to 40 wt.-%, even more preferably from 18 wt.-% to 35 wt.-%, still more preferably from 18 wt.-% to 30 wt.-%, yet more preferably from 20 wt.-% to 30 wt.-%, in each case based on the total weight of the pet food composition on dry-matter basis.

Suitable sources of protein for the pet food composition include animals and/or plants. For vegetarian animal compositions, the protein source will typically be comprised of 100 wt.-% plant protein, for example whey protein or albumin protein.

In a preferred embodiment, the pet food composition according to the invention contains carbohydrates other than D-allulose at a content within the range of from 18 wt.-% to 70 wt.-%, more preferably from 18 wt.-% to 65 wt.-%, even more preferably from 18 wt.-% to 60 wt.-%, still more preferably from 20 wt.-% to 70 wt.-%, yet more preferably from 30 wt.-% to 70 wt.-%, in each case based on the total weight of the pet food composition on dry-matter basis.

Typical carbohydrates other than D-allulose include but are not limited to monosaccharides such as glucose, fructose, galactose, and mannose; disaccharides such as sucrose, lactose, and maltose; and oligo- or polysaccharides such as starch and cellulose.

In a preferred embodiment, the pet food composition according to the invention contains monosaccharides other than D-allulose and disaccharides at a total content of at most 8.0 wt.-%, preferably at most 7.0 wt.-%, more preferably at most 6.0 wt.-%, still more preferably at most 5.0 wt.-%, yet more preferably at most 4.0 wt.-%, even more preferably at most 3.0 wt.-%, most preferably at most 2.0 wt.-%, and in particular at most 1.0 wt.-%, in each case based on the total content of monosaccharides and disaccharides (other than D-allulose) and based on the total weight of the pet food composition on dry-matter basis. However, this does not limit the possible content of oligosaccharides and/or polysaccharides such as starches. It has been surprisingly found that pet food compositions according to the invention not containing significant amounts of digestible mono- or disaccharides other than D-allulose such as glucose, fructose, sucrose and lactose are nonetheless well accepted by various pets due to the presence of D-allulose. Thus, the present invention provides well accepted pet food compositions that compared to conventional pet food compositions have health benefits with regard to calorie content.

Preferably, the pet food composition according to the invention contains
- fat at a content within the range of from 8.0 wt.-% to 32 wt.-%,
- protein at a content within the range of from 18 wt.-% to 50 wt.-%, and
- carbohydrate other than D-allulose at a content within the range of from 18 wt.-% to 74 wt.-%,
in each case based on the total weight of the pet food composition on dry-matter basis.

The pet food composition according to the invention may contain additives including but not limited to:
- N-containing compounds of non-proteinaceous nature, e.g. acid amides, amines, free amino acids, ammonium salts, alkaloids;
- ash including minerals (macro minerals such as calcium, phosphorus, sodium, chloride, potassium, magnesium, and microminerals, i.e., trace elements, such as iron, copper manganese, zinc, iodine, selenium) and further inorganic substances e.g. silicate;
- vitamins including vitamins A, B₁, B₂, B₆, B₁₂, C, D, E, pantothenic acid, niacin, biotin, folic acid, linoleic acid and choline.

Dry food may be prepared, e.g., by screw extrusion including cooking, shaping and cutting of raw ingredients into a specific kibble shape and size in a very short period of time, while simultaneously destroying detrimental micro-organisms. The ingredients may be mixed into homogenous expandable dough and cooked in an extruder (steam/pressure) and forced through a plate under pressure and high heat. After cooking, the kibbles are then allowed to cool, before optionally being sprayed with a coating which may include liquid fat or digest including liquid or powdered hydrolyzed forms of an animal tissue such as liver or intestine from, e.g., chicken or rabbit. Hot air drying then reduces the total moisture content to 10 wt.-% or less.

Typical components which provide the ingredients for a cat food composition, in addition to D-allulose, comprise beef, chicken meat, dried chicken liver, lamb meat, lamb liver, pork, turkey meat, turkey liver, poultry meal, fish meal, fowl protein hydrolysate, animal fats, plant oils, soy bean meal, pea bran, maize gluten, whole dry egg, ground corn, corn flour, rice, rice flour, dry sugar beet molasses, fructooligosaccharides, soluble fibers, plant gums, cellulose powder, clay, bakers yeast, iodized sodium chloride, calcium sulfate, sodium triphosphate, dicalcium phosphate, calcium carbonate, potassium chloride, choline chloride, magnesium oxide, zinc oxide, iron oxide, copper sulfate, iron sulfate, manganese oxide, calcium iodate, sodium selenite, provitamin D, thiamine, niacin, calcium pantothenate, pyridoxine hydrochloride, riboflavin, folic acid, biotin, vitamin B₁₂, taurine, L-carnitine, casein, D-methionine.

Typical components which provide the ingredients for a dog food composition, in addition to D-allulose, comprise e.g., chicken/beef/turkey, liver, broken pearl barley, ground corn, brute fat, whole dried egg, fowl protein hydrolysate, vegetable oil, calcium carbonate, choline chloride, potassium chloride, iodized salt, iron oxide, zinc oxide, copper sulfate, manganese oxide, sodium selenite, calcium iodate, provitamin D, vitamin B₁, niacin, calcium pantothenate, pyridoxin hydrochloride, riboflavin, folic acid, biotin, vitamin B₁₂.

Further components may, e.g. be omega-6-fatty acids, omega-3-fatty acids, L-carnitine, chondroitin sulfate, glucosamine, glutamine/glutamic acid, arginine, taurine and hydroxyproline.

In a preferred embodiment, the pet food composition according to the invention comprises an additive selected from the group consisting of preservatives, flavors, vitamins, mineral supplements, and combinations thereof.

Preferably, the pet food composition according to the invention has an additive content of at most 45 wt.-%, based on the total weight of the pet food composition on dry-matter basis.

In preferred embodiments, the pet food composition according to the invention is selected from the group consisting of complete pet food, complementary pet food, therapeutic/dietetic pet food and medicated pet food.

Complete pet foods are typically considered providing all the nutrients in the amounts and proportions animals need. Only complete pet foods are suitable for daily feeding without adding further food products except water. Nutritional standards for pet foods are provided by AAFCO (Association of Feed Control Officials) in the United States and by FEDIAF (European Pet Food Industry Federation) in Europe. Nutritional standards for pet foods already established or under development in countries outside the USA and Europe are largely based on AAFCO or FEDIAF recommendations.

Complementary pet food is typically designed to be only a part of the diet and they do not meet the known nutritional requirements when fed alone. They must be used in combination with additional food types to fulfil daily requirements.

Therapeutic pet food typically has a specific nutritional profile intended for therapeutic purposes such as support of cardiac, kidney or liver function. Due to the specific intended use and functionality, products have to comply with particular nutritional characteristics and requirements for their declaration. In Europe, the range of application is laid down in a specific regulation. In other regions, products typically have to be individually registered by the manufacturer and approved by the local veterinary authorities before they can be put on the market.

Medicated pet food (in contrast to dietetic pet food) typically contains veterinary drugs with a view either to restoring, correcting or modifying physiological functions by exerting a pharmacological, immunological or metabolic action, or to making a medical diagnosis.

The pet food composition according to the invention is typically sold in either dry form or wet canned form. A preferred type of packaging for the wet canned form of the pet food composition according to the invention is selected from the group consisting of aluminum cans, steel cans and foil pouches.

A dry form of the pet food composition according to the invention is typically made by extrusion cooking under high heat and pressure. Fat may then be sprayed on the food to increase palatability, and other minor ingredients, such as heat-sensitive vitamins, which would be destroyed in the extrusion process, may be added. The dry food according to the invention is typically packed in multi-wall paper bags, sometimes with a plastic film layer; similar bag styles with film laminates or coextrusions are also used.

A wet canned form of the pet food composition according to the invention may be prepared, e.g., by blending the raw ingredients. The mix is then fed into cans on a production line, the lids are sealed on and the filled cans are sterilized at a temperature of about 130 °C. for about 50 to 100 min.

In another preferred embodiment, the pet food composition according to the invention is a milk-product and/or a cereal product and/or a meat product and/or a fish product.

The pet food composition according to the invention can be typically classified based on the moisture content wherein a wet food preferably has a water content of at least 75 wt.-%, a semi-moist food preferably has a water content from 15 to 30 wt.-% and a dry food preferably has a water content from 6.0 to 10 wt.-%.

In a preferred embodiment, the pet food composition according to the invention is a wet pet food, or a semi-moist pet food, or a dry pet food.

Preferred wet pet food is selected form the group consisting of canned food, food in other closable packages such as bags, and compositions which are mixed with a liquid to obtain a wet food prior before feeding.

Preferred dry pet food is selected form the group consisting of seeds, grains, flakes, pellets, croquettes and mixtures thereof, as well as lick blocks, eatable toys for animals and structures designed to encourage natural instincts of a certain kind of animal, e.g. the rooting instinct in pigs. Further preferred dry food are compositions which are mixed with a liquid to obtain a wet food prior before feeding. More preferably, the dry food is selected from the group consisting of biscuits and dental chew treats, preferably for pets.

In another preferred embodiment, the pet food composition according to the invention is liquid. Non-limiting examples of liquid animal food according to the invention are beverages for new-born and young pets selected from puppies and kittens, as well as compositions which are mixed with a hot or cold liquid to obtain a beverage prior before feeding.

In a preferred embodiment of the pet food composition according to the invention, the food is in the form of a wet-type milk product, preferably for cats and/or dogs.

In a preferred embodiment of the pet food composition according to the invention, the pet food composition is a dry food which selected from the group consisting of candies, biscuits and dental chew treats for pets.

Preferably, the pet food composition according to the invention includes but is not limited to food for different weight classes of pets and pets in different stages of life.

According to the invention, preferred weight classes of pets are in the range of from 15 to 30 g, or from 25 to 40 g, or from 35 to 50 g, or from 45 to 70 g, or from 65 to 90 g, or from 85 to 120 g, or from 110 to 150 g, or from 140 to 250 g, or from 240 to 500 g, or from 450 to 1000 g. More preferred weight classes are in the range of from 0.8 kg to 10 kg, or from 10 kg to 50 kg, or from 50 kg to 100 kg, or from 100 to 150 kg.

According to the invention, preferred stages of life of pets are in the range of from 0 to 3 months, or from 3 month to 8 months, or from 8 to 20 months, or from 20 to 60 months, or from 60 to 144 months or from 144 to 240 months.

In a preferred embodiment, the pet food composition according to the invention is diet food for pets. Preferably, the diet food for pets is selected from the group consisting of vegetarian food, vegan food, low carb food, low fat food and food for animals having allergies, heart, liver, stomach and/or kidney problems.

In a preferred embodiment, the pet food composition according to the invention is used as pet food having reduced caloric content; wherein the reduction of caloric content is preferably relative to conventional pet food not containing D-allulose.

In a preferred embodiment, the pet food composition according to the invention is used as a dietetic pet food. For the purpose of the specification, the dietetic pet food pertains to diet or to regulation of the use of food; and/or is prepared or suitable for special diets, especially those requiring a restricted sugar or caloric intake.

In a preferred embodiment, the pet food composition according to the invention comprises a prebiotic in addition to D-allulose and/or a food ingredient for which an EU Health Claim has been acknowledged and/or a probiotic.

The content of said prebiotic, said food ingredient for which an EU Health Claim has been acknowledged, and said probiotic, respectively, is not particularly limited.

In another preferred embodiment, the pet food composition according to the invention is used for improving the balance of the intestinal flora.

A prebiotic is typically a selectively fermented ingredient that results in specific changes in the composition and/or activity of the gastrointestinal microbiota, thus conferring benefit(s) upon host health (Gibson et al. 2010. Food Science and Technology Bulletin: Functional Foods 7 (1), 1-19).

A prebiotic effect is scientifically known. A large number of human intervention studies have demonstrated that dietary consumption of prebiotic food products/ingredients/ supplements typically results in statistically significant changes in the composition of the fecal (and in some cases, the mucosal) gut microbiota. Most of the available data concern the selective stimulation of bifidobacteria (but also lactobacilli). It is recognized that such changes in the composition of the faecal microbiota, especially increase in bifidobacteria, can be regarded as a marker of intestinal health (M. Roberfroid et al., "Prebiotic effects: metabolic and health benefits", British Journal of Nutrition, 104(S2), 2010; S1-S51; R.R. Watson et al., "Probiotics, Prebiotics, and Synbiotics: Bioactive Foods in Health Promotion", Academic Press; 1st edition, 2015; and G.R. Gibson et al., "Handbook of Prebiotics", CRC Press; 1st edition 2008).

Prebiotics also seem to exert their nutritional benefits in various animal species, which by definition have an intestinal tract populated by a complex bacterial intestinal ecosystem. The concept typically remains valid, where the intestinal environment (pH, temperature, and digestive enzymes) and architecture (volume of different compartments, transit times, and villus structure) differs between the animal species. The beneficial consequences of prebiotics translate typically into improved zootechnical performance. Typically, productivity is increased and food conversion ratio decreased in feedstock. In pets, the risk for chronic disease (cancer, diabetes and obesity) is typically reduced and stool quality (odor and consistency) becomes typically improved (G.R. Gibson et al., "Handbook of Prebiotics", CRC Press; 1st edition 2008).

According to the invention, substances having prebiotic effects include but are not limited to certain fructooligosaccharides, galactooligosaccharides, isomaltooligosaccharides, xylooligosaccharides, soybean-oligosaccharides, cellobiose, lactulose, lactosucrose, raffinose, palatinose, stachyose, inosin, and inulin.

The *European Food Safety Authority* (EFSA) has issued scientific opinion on the substantiation of health claims for the following prebiotics: fructooligosaccharides, galactooligosaccharides, polydextrose, and wheat dextrin.

Also, the term "probiotic" is also well acknowledged in the art and for the purpose of the specification is to be understood with respect to animals, preferably vertebrates, more preferably mammals, still more preferably mammals selected from humans, pets and livestock. For the purpose of the specification, probiotics are preferably to be regarded as live microorganisms that, when administered in adequate amounts, confer a health benefit on the host (FAO/WHO 2001, as updated by Hill, et al, 2014. The International Scientific Association for Probiotics and Prebiotics consensus statement on the scope and appropriate use of the term probiotic. Nature Rev. Gastroenterol. Hepatol. 11:506-514).

Preferably, the probiotic which is preferably contained in the pet food composition according to the invention belongs to the bacteria and or molds and or yeasts selected from the group consisting of *Aspergillus*, *Bacillus*, *Bifidobacterium*, *Candida*, *Clostridium*, *Enterococcus*, *Lactobacillus*, *Leuconostoc*, *Pediococcus, Saccharomyces*, *Streptococcus* and *Torulopsis.*

Preferably, said probiotic is selected from the group consisting of *Aspergillus oryzae*, *Bacillus subtilis, Bacillus cereus, Bacillus toyoi, Bacillus natto, Bacillus mesentericus, Bacillus lichenformis*, *Bifidobacterium bifidum, Bifidobacterium longum*/*animalis, Bifidobacterium pseudolongum, Bifidobacterium breve*/*infantis, Bifidobacterium thermophilum, Candida pintolopesii, Clostridium butyricum, Enterococcus faecium, Enterococcus faecalis, Lactobacillus delbruecki ssp. bulgaricus, Lactobacillus acidophilus, Lactobacillus casei*/*paracasei, Lactobacillus rhamnosus, Lactobacillus reuteri, Lactobacillus plantarum, Lactobacillus farciminis, Lactobacillus fermentum, Lactobacillus brevis, Lactobacillus helveticus, Lactobacillus lactis, Lactobacillus murinus, Leuconostoc mesenteroides, Pediococcus cerevisae, Pediococcus acidilactici, Pediococcus pentosaceus, Saccharomyces cerivisae, Saccharomyces fragilis, Streptococcus salivarius ssp. thermophilus, Streptococcus lactis, Streptococcus infantarius* and *Torulopsis spp.*

The pet food composition according to the invention also contains carbohydrates other than D-allulose.

In a preferred embodiment of the pet food composition according to the invention, the total content of carbohydrates other than D-allulose is at most 5.0 wt.-%, preferably at most 2.5 wt.-%, more preferably at most 0.5 wt.-%, based on the total weight of the pet food composition on dry matter basis.

In another preferred embodiment of the pet food composition according to the invention, the caloric value of the pet food is at most 6000 kcal/kg, preferably at most 5500 kcal/kg, more preferably at most 5000 kcal/kg, still more preferably at most 4500 kcal/kg, yet more preferably at most 4000 kcal/kg, even more preferably at most 3500 kcal/kg, most preferably at most 3000 kcal/kg, and in particular at most 2500 kcal/kg, in each case based on the total weight of the pet food composition on dry-matter basis.

In still another preferred embodiment of the pet food composition according to the invention, the pet food composition is selected from the group consisting of regular food, organic food, vegetarian food, vegan food, low carb food, low fat food, low protein food, low carb/low fat food, low carb/high fat food, low carb/low protein food, low carb/high protein food, low fat/low protein food, and high carb food.

Preferably, the pet food composition according to the invention comprises at most 5.0 wt.-% of L-allulose, relative to the total content of D-allulose contained in the pet food composition, preferably essentially no L-allulose.

Preferably, the pet food composition according to the invention comprises one or more mono- and/or disaccharides other than D-allulose, preferably wherein the content of D-allulose is greater than the total weight content of said one or more mono- and/or disaccharides other than D-allulose.

Preferred embodiments E1 to E24 of the pet food composition according to the invention are compiled in the following table:

| [wt.-%]¹ | E1 | E2 | E3 | E4 | E5 | E6 |
|---|---|---|---|---|---|---|
| D-allulose | 0.05 to 4.5 | 0.05 to 4.5 | 0.075 to 4.0 | 0.075 to 4.0 | 0.1 to 3.5 | 0.1 to 3.0 |
| fat | 8.0 to 30 | 8.0 to 25 | 10 to 32 | 10 to 30 | 15 to 30 | 15 to 30 |
| protein | 18 to 45 | 18 to 40 | 18 to 35 | 18 to 30 | 20 to 30 | 20 to 30 |
| carbohydrates other than D-allulose | 18 to 70 | 18 to 65 | 16 to 60 | 20 to 70 | 30 to 70 | 30 to 70 |
| | | | | | | |

| | E7 | E8 | E9 | E10 | E11 | E12 |
|---|---|---|---|---|---|---|
| D-allulose | 1.0±0.8 | 1.0±0.7 | 1.0±0.6 | 1.0±0.5 | 1.0±0.4 | 1.0±0.3 |
| fat | 19±10 | 19±9 | 19±8 | 19±7 | 19±6 | 19±5 |
| protein | 31±12 | 31±11 | 31±10 | 31±9 | 31±8 | 31±7 |
| carbohydrates other than D-allulose | 49±23 | 49±20 | 49±17 | 49±15 | 49±13 | 49±11 |
| | | | | | | |

| | E13 | E14 | E15 | E16 | E17 | E18 |
|---|---|---|---|---|---|---|
| D-allulose | 2.0±1.8 | 2.0±1.6 | 2.0±1.4 | 2.0±1.2 | 2.0±1.0 | 2.0±0.8 |
| fat | 19±10 | 19±9 | 19±8 | 19±7 | 19±6 | 19±5 |
| protein | 31±12 | 31±11 | 31±10 | 31±9 | 31±8 | 31±7 |
| carbohydrates other than D-allulose | 48±23 | 48±20 | 48±17 | 48±15 | 48±13 | 48±11 |
| | | | | | | |

| | E19 | E20 | E21 | E22 | E23 | E24 |
|---|---|---|---|---|---|---|
| D-allulose | 3.0±2.8 | 3.0±2.4 | 3.0±2.0 | 3.0±1.6 | 3.0±1.2 | 3.0±0.8 |
| fat | 19±10 | 19±9 | 19±8 | 19±7 | 19±6 | 19±5 |
| protein | 31±12 | 31±11 | 31±10 | 31±9 | 31±8 | 31±7 |
| carbohydrates other than D-allulose | 47±23 | 47±20 | 47±17 | 47±15 | 47±13 | 47±11 |

| | | | | | | |
|---|---|---|---|---|---|---|
| ¹ in each case based on the total weight of the pet food composition on dry-matter basis | | | | | | |

Another aspect of the invention relates to the use of the pet food composition according to the invention for the prevention or treatment of a condition in pets, wherein the condition is selected from the group consisting of impaired glucose tolerance, type 2 diabetes mellitus, hyperlipidemia, a metabolic syndrome, and obesity, wherein the pet is preferably a cat or a dog.

Another aspect of the invention relates to the use of D-allulose in the manufacture of a pet food composition for weight reduction in cats and dogs.

A further aspect of the invention relates to the use of the pet food composition according to the invention as cat food.

Still another aspect of the invention relates to the use of the pet food composition according to the invention as dog food.

Yet another aspect of the invention relates to the use of the pet food composition according to the invention for improving the balance of the intestinal flora.

The following examples illustrate the invention but are not to be construed as limiting its scope.

### EXAMPLE 1

A typical wet food for adult dogs may, e.g. comprise, from 0.025 wt.-% to 5 wt.-% D-allulose, 23 wt.-% protein, 14 wt.-% fat, 48 wt.-% starch, 0.8 wt.-% fiber, 2 wt.-% linoleic acid, 0.6 wt.-% calcium, 0.5 wt.-% phosphorus, the Ca:P ratio being 1:1, 0.2 wt.-% potassium, 0.6 wt.-% sodium, 0.09 wt.-% chloride, 0.09 wt.-% magnesium, 170 mg/kg of iron, 15 mg/kg of copper, 70 mg/kg of manganese, 220 mg/kg of zinc, 4 mg/kg of iodine, 0.43 mg/kg of selenium, 74000 IU/kg of vitamin A, 1200 IU/kg of vitamin D, 11 mg/kg of vitamin B₁, 6 mg/kg of riboflavin, 30 mg/kg of pantothenic acid, 20 mg/kg of niacin, 4.3 mg/kg of pyridoxine, 0.9 mg/kg of folic acid, 0.2 µg/kg of vitamin B₁₂, 2500 mg/kg of choline, 0.8 mg/kg biotin, 2500 mg/kg choline, all percentages being based on dry weight of the total food composition.

### EXAMPLE 2

For example, a typical wet food for adult dogs may, e.g. comprise, 0.05 wt.-% D-allulose, 24 wt.-% protein, 14 wt.-% fat, 51 wt.-% starch, 0.8 wt.-% fiber, 3 wt.-% linoleic acid, 0.6 wt.-% calcium, 0.5 wt.-% phosphorus, the Ca:P ratio being 1:1, 0.2 wt.-% potassium, 0.6 wt.-% sodium, 0.09 wt.-% chloride, 0.09 wt.-% magnesium, 170 mg/kg of iron, 15 mg/kg of copper, 70 mg/kg of manganese, 220 mg/kg of zinc, 4 mg/kg of iodine, 0.43 mg/kg of selenium, 74000 IU/kg of vitamin A, 1200 IU/kg of vitamin D, 11 mg/kg of vitamin B₁, 6 mg/kg of riboflavin, 30 mg/kg of pantothenic acid, 20 mg/kg of niacin, 4.3 mg/kg of pyridoxine, 0.9 mg/kg of folic acid, 0.2 µg/kg of vitamin B₁₂, 2500 mg/kg of choline, 0.8 mg/kg biotin, 2500 mg/kg choline, all percentages being based on dry weight of the total food composition.

### EXAMPLE 3

As another example, a typical wet food for adult dogs may, e.g. comprise, 2.8 wt.-% D-allulose, 24 wt.-% protein, 16 wt.-% fat, 49 wt.-% starch, 0.8 wt.-% fiber, 2 wt.-% linoleic acid, 0.6 wt.-% calcium, 0.5 wt.-% phosphorus, the Ca:P ratio being 1:1, 0.2 wt.-% potassium, 0.6 wt.-% sodium, 0.09 wt.-% chloride, 0.09 wt.-% magnesium, 170 mg/kg of iron, 15 mg/kg of copper, 70 mg/kg of manganese, 220 mg/kg of zinc, 4 mg/kg of iodine, 0.43 mg/kg of selenium, 74000 IU/kg of vitamin A, 1200 IU/kg of vitamin D, 11 mg/kg of vitamin B₁, 6 mg/kg of riboflavin, 30 mg/kg of pantothenic acid, 20 mg/kg of niacin, 4.3 mg/kg of pyridoxine, 0.9 mg/kg of folic acid, 0.2 µg/kg of vitamin B₁₂, 2500 mg/kg of choline, 0.8 mg/kg biotin, 2500 mg/kg choline, all percentages being based on dry weight of the total food composition.

### EXAMPLE 4

A typical wet food for adult cats may, e.g. comprise, 0.05 wt.-% D-allulose, 44 wt.-% protein, 25 wt.-% fat, 20 wt.-% starch, 2.5 wt.-% fiber, 0.8 wt.-% calcium, 0.6 wt.-% phosphorus, 0.8 wt.-% potassium, 0.3 wt.-% sodium, 0.09 wt.-% chloride, 0.08 wt.-% magnesium, 0.25 wt.-% taurine, 170 mg/kg of iron, 15 mg/kg of copper, 70 mg/kg of manganese, 220 mg/kg of zinc, 4 mg/kg of iodine, 0.43 mg/kg of selenium, 74000 IU/kg of vitamin A, 1200 IU/kg of vitamin D, 11 mg/kg of vitamin B₁, 6 mg/kg of riboflavin, 30 mg/kg of pantothenic acid, 20 mg/kg of niacin, 4.3 mg/kg of pyridoxine, 0.9 mg/kg of folic acid, 0.2 µg/kg of vitamin B₁₂, 2500 mg/kg of choline, 0.8 mg/kg biotin, 2500 mg/kg choline, all percentages being based on dry weight of the total food composition.

### EXAMPLE 5

Another typical wet food for adult cats may, e.g. comprise, 2.5 wt.-% D-allulose, 43.5 wt.-% protein, 24 wt.-% fat, 19.5 wt.-% starch, 2.4 wt.-% fiber, 0.7 wt.-% calcium, 0.6 wt.-% phosphorus, 0.8 wt.-% potassium, 0.25 wt.-% sodium, 0.09 wt.-% chloride, 0.08 wt.-% magnesium, 0.25 wt.-% taurine, 170 mg/kg of iron, 15 mg/kg of copper, 70 mg/kg of manganese, 220 mg/kg of zinc, 4 mg/kg of iodine, 0.43 mg/kg of selenium, 74000 IU/kg of vitamin A, 1200 IU/kg of vitamin D, 11 mg/kg of vitamin B₁, 6 mg/kg of riboflavin, 30 mg/kg of pantothenic acid, 20 mg/kg of niacin, 4.3 mg/kg of pyridoxine, 0.9 mg/kg of folic acid, 0.2 µg/kg of vitamin B₁₂, 2500 mg/kg of choline, 0.8 mg/kg biotin, 2500 mg/kg choline, all percentages being based on dry weight of the total food composition.

### EXAMPLE 6

Exemplarily, a typical wet food for adult cats may, e.g. comprise, 4.85 wt.-% D-allulose, 44 wt.-% protein, 21 wt.-% fat, 19 wt.-% starch, 2.5 wt.-% fiber, 0.8 wt.-% calcium, 0.6 wt.-% phosphorus, 0.8 wt.-% potassium, 0.3 wt.-% sodium, 0.09 wt.-% chloride, 0.08 wt.-% magnesium, 0.25 wt.-% taurine, 170 mg/kg of iron, 15 mg/kg of copper, 70 mg/kg of manganese, 220 mg/kg of zinc, 4 mg/kg of iodine, 0.43 mg/kg of selenium, 74000 IU/kg of vitamin A, 1200 IU/kg of vitamin D, 11 mg/kg of vitamin B₁, 6 mg/kg of riboflavin, 30 mg/kg of pantothenic acid, 20 mg/kg of niacin, 4.3 mg/kg of pyridoxine, 0.9 mg/kg of folic acid, 0.2 µg/kg of vitamin B₁₂, 2500 mg/kg of choline, 0.8 mg/kg biotin, 2500 mg/kg choline, all percentages being based on dry weight of the total food composition.

### EXAMPLE 7

A typical dry food for adult dogs may, e.g. comprise, from 0.025 wt.-% to 5 wt.-% D-allulose, 25 wt.-% protein, 12 wt.-% fat, 41.5 wt.-% starch, 2.5 wt.-% fiber, 1 wt.-% linoleic acid, 1 wt.-% calcium, 0.8 wt.-% phosphorus, the Ca:P ratio being 1:1, 0.6 wt.-% potassium, 0.35 wt.-% sodium, 0.09 wt.-% chloride, 0.1 wt.-% magnesium, 170 mg/kg of iron, 35 mg/kg of copper, 70 mg/kg of manganese, 220 mg/kg of zinc, 4 mg/kg of iodine, 0.43 mg/kg of selenium, 15000 IU/kg of vitamin A, 1200 IU/kg of vitamin D, 11 mg/kg of vitamin B₁, 6 mg/kg of riboflavin, 30 mg/kg of pantothenic acid, 20 mg/kg of niacin, 4.3 mg/kg of pyridoxine, 0.9 mg/kg of folic acid, 0.2 µg/kg of vitamin B₁₂, 2500 mg/kg of choline, 0.8 mg/kg biotin, all percentages being based on dry weight of the total food composition.

### EXAMPLE 8

Another typical dry food for adult dogs may, e.g. comprise, 1.5 wt.-% D-allulose, 25 wt.-% protein, 12 wt.-% fat, 41.5 wt.-% starch, 2.5 wt.-% fiber, 1 wt.-% linoleic acid, 1 wt.-% calcium, 0.8 wt.-% phosphorus, the Ca:P ratio being 1:1, 0.6 wt.-% potassium, 0.35 wt.-% sodium, 0.09 wt.-% chloride, 0.1 wt.-% magnesium, 170 mg/kg of iron, 35 mg/kg of copper, 70 mg/kg of manganese, 220 mg/kg of zinc, 4 mg/kg of iodine, 0.43 mg/kg of selenium, 15000 IU/kg of vitamin A, 1200 IU/kg of vitamin D, 11 mg/kg of vitamin B₁, 6 mg/kg of riboflavin, 30 mg/kg of pantothenic acid, 20 mg/kg of niacin, 4.3 mg/kg of pyridoxine, 0.9 mg/kg of folic acid, 0.2 µg/kg of vitamin B₁₂, 2500 mg/kg of choline, 0.8 mg/kg biotin, all percentages being based on dry weight of the total food composition.

### EXAMPLE 9

Alternatively, a typical dry food for adult dogs may, e.g. comprise, 5 wt.-% D-allulose, 25 wt.-% protein, 12 wt.-% fat, 41.5 wt.-% starch, 2.5 wt.-% fiber, 1 wt.-% linoleic acid, 1 wt.-% calcium, 0.8 wt.-% phosphorus, the Ca:P ratio being 1:1, 0.6 wt.-% potassium, 0.35 wt.-% sodium, 0.09 wt.-% chloride, 0.1 wt.-% magnesium, 170 mg/kg of iron, 35 mg/kg of copper, 70 mg/kg of manganese, 220 mg/kg of zinc, 4 mg/kg of iodine, 0.43 mg/kg of selenium, 15000 IU/kg of vitamin A, 1200 IU/kg of vitamin D, 11 mg/kg of vitamin B₁, 6 mg/kg of riboflavin, 30 mg/kg of pantothenic acid, 20 mg/kg of niacin, 4.3 mg/kg of pyridoxine, 0.9 mg/kg of folic acid, 0.2 µg/kg of vitamin B₁₂, 2500 mg/kg of choline, 0.8 mg/kg biotin, all percentages being based on dry weight of the total food composition.

### EXAMPLE 10

Another typical dry food for adult dogs may, e.g. comprise, 0.9 wt.-% D-allulose, 25 wt.-% protein, 12 wt.-% fat, 41.5 wt.-% starch, 2.5 wt.-% fiber, 1 wt.-% linoleic acid, 1 wt.-% calcium, 0.8 wt.-% phosphorus, the Ca:P ratio being 1:1, 0.6 wt.-% potassium, 0.35 wt.-% sodium, 0.09 wt.-% chloride, 0.1 wt.-% magnesium, 170 mg/kg of iron, 35 mg/kg of copper, 70 mg/kg of manganese, 220 mg/kg of zinc, 4 mg/kg of iodine, 0.43 mg/kg of selenium, 15000 IU/kg of vitamin A, 1200 IU/kg of vitamin D, 11 mg/kg of vitamin B₁, 6 mg/kg of riboflavin, 30 mg/kg of pantothenic acid, 20 mg/kg of niacin, 4.3 mg/kg of pyridoxine, 0.9 mg/kg of folic acid, 0.2 µg/kg of vitamin B₁₂, 2500 mg/kg of choline, 0.8 mg/kg biotin, all percentages being based on dry weight of the total food composition.

### EXAMPLE 11

A typical dry food for adult cats may, e.g. comprise, 0.025 wt.-% to 5 wt.-% D-allulose, 32 wt.-% protein, 14 wt.-% fat, 25.5 wt.-% starch, 7 wt.-% dietetic fibers, 4.1 wt.-% fiber, 2.9 wt.-% linoleic acid, 0.08 wt.-% arachidonic acid, 0.15 wt.-% taurine, 50 mg/kg L-carnitine, 1 wt.-% calcium, 0.8 wt.-% phosphorus, the Ca:P ratio being at least 1:1, 0.5 wt.-% potassium, 0.4 wt.-% sodium, 0.6 wt.-% chloride, 0.08 wt.-% magnesium, 190 mg/kg of iron, 30 mg/kg of copper, 60 mg/kg of manganese, 205 mg/kg of zinc, 2.5 mg/kg of iodine, 0.2 mg/kg of selenium, 25000 IU/kg of vitamin A, 1500 IU/kg of vitamin D, 20 mg/kg of vitamin B1, 40 mg/kg of riboflavin, 56 mg/kg of pantothenic acid, 153 mg/kg of niacin, 14 mg/kg of pyridoxine, 3.2 mg/kg of folic acid, 0.2 mg/kg of vitamin B₁₂, 1 mg/kg of biotin, 3000 mg/kg of choline, all percentages being based on dry weight of the total food composition.

### EXAMPLE 12

Another typical dry food for adult cats may, e.g. comprise, 2.25 wt.-% D-allulose, 31 wt.-% protein, 16 wt.-% fat, 24.5 wt.-% starch, 9 wt.-% dietetic fibers, 3.5 wt.-% fiber, 3.2 wt.-% linoleic acid, 0.08 wt.-% arachidonic acid, 0.15 wt.-% taurine, 50 mg/kg L-carnitine, 1 wt.-% calcium, 0.8 wt.-% phosphorus, the Ca:P ratio being at least 1:1, 0.6 wt.-% potassium, 0.4 wt.-% sodium, 0.6 wt.-% chloride, 0.08 wt.-% magnesium, 190 mg/kg of iron, 30 mg/kg of copper, 60 mg/kg of manganese, 205 mg/kg of zinc, 2.5 mg/kg of iodine, 0.2 mg/kg of selenium, 25000 IU/kg of vitamin A, 1500 IU/kg of vitamin D, 20 mg/kg of vitamin B1, 40 mg/kg of riboflavin, 56 mg/kg of pantothenic acid, 153 mg/kg of niacin, 14 mg/kg of pyridoxine, 3.2 mg/kg of folic acid, 0.2 mg/kg of vitamin B₁₂, 1 mg/kg of biotin, 3000 mg/kg of choline, all percentages being based on dry weight of the total food composition.

### EXAMPLE 13

A further typical dry food for adult cats may, e.g. comprise, 4.75 wt.-% D-allulose, 28 wt.-% protein, 16 wt.-% fat, 24.5 wt.-% starch, 11 wt.-% dietetic fibers, 4.5 wt.-% fiber, 3.4 wt.-% linoleic acid, 0.08 wt.-% arachidonic acid, 0.15 wt.-% taurine, 50 mg/kg L-carnitine, 1 wt.-% calcium, 0.8 wt.-% phosphorus, the Ca:P ratio being at least 1:1, 0.6 wt.-% potassium, 0.4 wt.-% sodium, 0.6 wt.-% chloride, 0.08 wt.-% magnesium, 190 mg/kg of iron, 30 mg/kg of copper, 60 mg/kg of manganese, 205 mg/kg of zinc, 2.5 mg/kg of iodine, 0.2 mg/kg of selenium, 25000 IU/kg of vitamin A, 1500 IU/kg of vitamin D, 20 mg/kg of vitamin B1, 40 mg/kg of riboflavin, 56 mg/kg of pantothenic acid, 153 mg/kg of niacin, 14 mg/kg of pyridoxine, 3.2 mg/kg of folic acid, 0.2 mg/kg of vitamin B₁₂, 1 mg/kg of biotin, 3000 mg/kg of choline, all percentages being based on dry weight of the total food composition.

### EXAMPLE 14

An additional typical dry food for adult cats may, e.g. comprise, 0.75 wt.-% D-allulose, 32 wt.-% protein, 15 wt.-% fat, 25.5 wt.-% starch, 8 wt.-% dietetic fibers, 4.5 wt.-% fiber, 2.4 wt.-% linoleic acid, 0.08 wt.-% arachidonic acid, 0.15 wt.-% taurine, 50 mg/kg L-carnitine, 0.8 wt.-% calcium, 0.8 wt.-% phosphorus, the Ca:P ratio being at least 1:1, 0.6 wt.-% potassium, 0.4 wt.-% sodium, 0.6 wt.-% chloride, 0.08 wt.-% magnesium, 190 mg/kg of iron, 30 mg/kg of copper, 60 mg/kg of manganese, 205 mg/kg of zinc, 2.5 mg/kg of iodine, 0.2 mg/kg of selenium, 25000 IU/kg of vitamin A, 1500 IU/kg of vitamin D, 20 mg/kg of vitamin B1, 40 mg/kg of riboflavin, 56 mg/kg of pantothenic acid, 153 mg/kg of niacin, 14 mg/kg of pyridoxine, 3.2 mg/kg of folic acid, 0.2 mg/kg of vitamin B₁₂, 1 mg/kg of biotin, 3000 mg/kg of choline, all percentages being based on dry weight of the total food composition.

### EXAMPLE 15

A typical wet-type milk food for cats may, e.g. comprise, from 0.025 wt.-% to 5 wt.-% D-allulose, 5 wt.-% raw protein, 2 wt.-% raw fat, 0.5 wt.-% to 0.3 wt.-% ashes, at minimum 80 wt.-% moisture, 0.8 wt.-% raw starch, vitamins A, E, B₁, B₂, B₆ and/or B₁₂ in a range of from 0.001 mg to about 5 mg per kg food, wherein all percentages being based on dry weight of the total food composition.

### EXAMPLE 16

Accordingly, a typical wet-type milk food for cats may, e.g. comprise, 0.025 wt.-% D-allulose, 5 wt.-% raw protein, 2 wt.-% raw fat, 0.5 wt.-% to 0.3 wt.-% ashes, at minimum 80 wt.-% moisture, 0.8 wt.-% raw starch, vitamins A, E, B₁, B₂, B₆ and/or B₁₂ in a range of from 0.001 mg to about 5 mg per kg food, wherein all percentages being based on dry weight of the total food composition.

### EXAMPLE 17

Another typical wet-type milk food for cats may, e.g. comprise, 2.9 wt.-% D-allulose, 5 wt.-% raw protein, 2 wt.-% raw fat, 0.5 wt.-% to 0.3 wt.-% ashes, at minimum 80 wt.-% moisture, 0.8 wt.-% raw starch, vitamins A, E, B₁, B₂, B₆ and/or B₁₂ in a range of from 0.001 mg to about 5 mg per kg food, wherein all percentages being based on dry weight of the total food composition.

### EXAMPLE 18

A further typical wet-type milk food for cats may, e.g. comprise, 4.95 wt.-% D-allulose, 5 wt.-% raw protein, 2 wt.-% raw fat, 0.5 wt.-% to 0.3 wt.-% ashes, at minimum 80 wt.-% moisture, 0.8 wt.-% raw starch, vitamins A, E, B₁, B₂, B₆ and/or B₁₂ in a range of from 0.001 mg to about 5 mg per kg food, wherein all percentages being based on dry weight of the total food composition.

### EXAMPLE 19

To a cat milk comprising 0.03 wt.-% arachidonic acid, 1.9 wt.-% arginine, 1.0 mg/kg biotin, 0.5 wt.-% chloride, 3000.0 mg/kg choline, 1.2 wt.-% DL-methionine, 0.5 wt.-% fiber, 0.13 wt.-% eicosapentaenoic acid/docosahexaenoic acid (EPA/DHA), 100.0 mg/kg iron, 30.0 wt.-% fat, 3.0 wt.-% moisture, 3.2 mg/kg folic acid, 4.0 mg/kg iodine, 0.67 wt.-% potassium, 1.1 wt.-% calcium, 15.0 mg/kg copper, 2.44 wt.-% L-lysin, 10.0 wt.-% lactose, 2.4 wt.-% linoleic acid, 0.1 wt.-% magnesium, 80.0 mg/kg manganese, 0.17 wt.-% sodium, 0.5 wt.-% omega 3 fatty acids, 2.4 wt.-% omega 6 fatty acids, 0.8 wt.-% phosphorus, 30.0 wt.-% protein, 5.0 wt.-% ash, 0.43 mg/kg selenium, 0.22 wt.-% taurine, 25000.0 UI/kg vitamin A, 20.0 mg/kg vitamin B₁, 0.3 mg/kg vitamin B₁₂, 40.0 mg/kg vitamin B₂, 155.0 mg/kg vitamin B₃, 56.0 mg/kg vitamin B₅, 15.0 mg/kg vitamin B₆, 300.0 mg/kg Vitamin C, 1500.0 UI/kg vitamin D₃, 600 mg/kg vitamin E and 190.0 mg/kg zinc, D-allulose in aqueous solution and in an amount sufficient to provide 0.025 wt.-% to 5.0 wt.-% of D-allulose in the final food composition is added.

### EXAMPLE 20

To a Crispy Cat Candy Bar (as supplied by Tree Huggin' Treats) D-allulose in aqueous solution and in an amount sufficient to provide 0.025 wt.-% to 5.0 wt.-% of D-allulose in the final food composition is added (fat content 35 wt.-%, carbohydrate content 39 wt.-%, and protein content 26 wt.-%).

### EXAMPLE 21

To a typical dog treat comprising 10 wt.-% water, 24 wt.-% protein, 6 wt.-% fat, 27.5 wt.-% starch, 6.5 wt.-% ash, 2.5 wt.-% fiber, 1.2 wt.-% calcium, 0.8 wt.-% phosphorus and 0.25 wt.-% sodium, D-allulose is added in aqueous solution and in an amount sufficient to provide 0.025 wt.-% to 5.0 wt.-% of D-allulose in the final food composition.

### EXAMPLE 22

To a typical dog treat comprising 10 wt.-% water, 24 wt.-% protein, 6 wt.-% fat, 27.5 wt.-% starch, 6.5 wt.-% ash, 2.5 wt.-% fiber, 1.2 wt.-% calcium, 0.8 wt.-% phosphorus and 0.25 wt.-% sodium, D-allulose is added in aqueous solution and in an amount sufficient to provide 4.3 wt.-% of D-allulose in the final food composition.

### EXAMPLE 23

To another typical dog treat comprising 10 wt.-% water, 20 wt.-% protein, 10 wt.-% fat, 27.0 wt.-% starch, 7.0 wt.-% ash, 2.5 wt.-% fiber, 1.2 wt.-% calcium, 0.8 wt.-% phosphorus and 0.25 wt.-% sodium, D-allulose is added in aqueous solution and in an amount sufficient to provide 0.25 wt.-% of D-allulose in the final food composition.

### EXAMPLE 24

To still another typical dog treat comprising 12 wt.-% water, 22 wt.-% protein, 12 wt.-% fat, 21.5 wt.-% starch, 5.0 wt.-% ash, 4.5 wt.-% fiber, 0.8 wt.-% calcium, 0.8 wt.-% phosphorus and 0.25 wt.-% sodium, D-allulose is added in aqueous solution and in an amount sufficient to provide 2.2 wt.-% of D-allulose in the final food composition.

### EXAMPLE 25

To a typical cat treat comprising 39.9 wt.-% protein, 13.6 wt.-% fat, 17.6 wt.-% fiber, 0.99 wt.-% calcium, 0.75 wt.-% potassium, 0.09 wt.-% magnesium, 0.35 wt.-% sodium, 0.75 wt.-% phosphorus, 2513 mg/kg taurine, 10030 IU/kg vitamin A, 129 mg/kg vitamin C, 979 IU/kg vitamin D, 857 mg/kg vitamin E, 561 mg/kg L-carnitine, 2.3 wt.-% L-lysine, 1.48 wt.-% omega-3-fatty acids, 2.4 wt.-% omega-6-fatty acids and 2.1 mg/kg β-carotene, D-allulose is added to provide 2.4 wt.-% of D-allulose in the final food composition, in each case based on the total weight of the pet food composition on dry-matter basis.

### EXAMPLE 26

Another typical cat treat comprising 39.9 wt.-% protein, 13.6 wt.-% fat, 17.6 wt.-% fiber, 0.99 wt.-% calcium, 0.75 wt.-% potassium, 0.09 wt.-% magnesium, 0.35 wt.-% sodium, 0.75 wt.-% phosphorus, 2513 mg/kg taurine, 10030 IU/kg vitamin A, 129 mg/kg vitamin C, 979 IU/kg vitamin D, 857 mg/kg vitamin E, 561 mg/kg L-carnitine, 2.3 wt.-% L-lysine, 1.48 wt.-% omega-3-fatty acids, 2.4 wt.-% omega-6-fatty acids and 2.1 mg/kg β-carotene, contains 4.95 wt.-% of D-allulose in the final food composition, in each case based on the total weight of the pet food composition on dry-matter basis.

### EXAMPLE 27

An alternative cat treat comprising 39.9 wt.-% protein, 13.6 wt.-% fat, 17.6 wt.-% fiber, 0.99 wt.-% calcium, 0.75 wt.-% potassium, 0.09 wt.-% magnesium, 0.35 wt.-% sodium, 0.75 wt.-% phosphorus, 2513 mg/kg taurine, 10030 IU/kg vitamin A, 129 mg/kg vitamin C, 979 IU/kg vitamin D, 857 mg/kg vitamin E, 561 mg/kg L-carnitine, 2.3 wt.-% L-lysine, 1.48 wt.-% omega-3-fatty acids, 2.4 wt.-% omega-6-fatty acids and 2.1 mg/kg β-carotene, contains 15.8 wt.-% of D-allulose in the final food composition, in each case based on the total weight of the pet food composition on dry-matter basis.

### EXAMPLE 28

To a digestive care cat food composition comprising 0.06 wt.-% arachidonic acid, 1.94 wt.-% arginine, 1.65 mg/kg biotin, 0.72 wt.-% chloride, 3200.0 mg/kg choline, 0.71 wt.-% DL-methionine, 3.3 wt.-% fiber, 0.32 wt.-% icosapentaenoic acid/docosahexaenoic acid (EPA/DHA), 163.0 mg/kg iron, 10.0 wt.-% fat, 4.5 wt.-% moisture, 4.2 mg/kg folic acid, 4.6 mg/kg iodine, 0.7 wt.-% potassium, 0.85 wt.-% calcium, 15.0 mg/kg copper, 1.25 wt.-% L-lysin, 2.35 wt.-% linoleic acid, 0.06 wt.-% magnesium, 71.0 mg/kg manganese, 0.6 wt.-% sodium, 0.79 wt.-% omega-3 fatty acids, 2.32 wt.-% omega-6 fatty acids, 0.79 wt.-% phosphorus, 28.0 wt.-% protein, 4.5 wt.-% ash, 0.41 mg/kg selenium, 24.7 wt.-% starch, 1000 mg/kg taurine, 20000.0 UI/kg vitamin A, 15.7 mg/kg vitamin B₁, 0.16 mg/kg vitamin B₁₂, 56.4 mg/kg vitamin B₂, 174.6 mg/kg vitamin B₃, 52.5 mg/kg vitamin B₅, 25.8 mg/kg vitamin B₆, 300.0 mg/kg Vitamin C, 1100.0 UI/kg vitamin D₃, 600 mg/kg vitamin E and 201.0 mg/kg zinc, D-allulose in aqueous solution and in an amount sufficient to provide 0.025 wt.-% to 5.0 wt.-% of D-allulose in the final food composition is added.

### EXAMPLE 29

To a joint/bone supporting and digestive care food for adult dogs composition comprising 1.5 wt.-% arginine, 2.75 mg/kg biotin, 0.51 wt.-% chloride, 2000.0 mg/kg choline, 0.71 wt.-% DL-methionine, 0.3 wt.-% icosapentaenoic acid/docosahexaenoic acid (EPA/DHA), 206.0 mg/kg iron, 15.0 wt.-% fat, 5.5 wt.-% moisture, 9.0 mg/kg folic acid, 990 mg/kg glucosamine chloride, 5.3 mg/kg iodine, 0.7 wt.-% potassium, 0.8 wt.-% calcium, 15.0 mg/kg copper, 1.1 wt.-% L-lysin, 2.54 wt.-% linoleic acid, 5.0 mg/kg lutein, 0.08 wt.-% magnesium, 78.0 mg/kg manganese, 0.3 wt.-% sodium, 0.66 wt.-% omega-3 fatty acids, 2.49 wt.-% omega-6 fatty acids, 0.7 wt.-% phosphorus, 21.0 wt.-% protein, 4.5 wt.-% ash, 1.8 wt.-% fiber, 0.28 mg/kg selenium, 30.8 wt.-% starch, 0.2 wt.-% taurine, 29000.0 UI/kg vitamin A, 4.2 mg/kg vitamin B₁, 0.07 mg/kg vitamin B₁₂, 3.8 mg/kg vitamin B₂, 15 mg/kg vitamin B₃, 34.4 mg/kg vitamin B₅, 26.4 mg/kg vitamin B₆, 200.0 mg/kg Vitamin C, 1000.0 UI/kg vitamin D₃, 600 mg/kg vitamin E and 225.0 mg/kg zinc, D-allulose in aqueous solution and in an amount sufficient to provide 0.025 wt.-% to 5.0 wt.-% of D-allulose in the final food composition is added.

## Claims

1. A pet food composition containing
- D-allulose at a content of at least 0.025 wt.-%, and
- fat at a content within the range of from 0.1 wt.-% to 48 wt.-%, and
- protein at a content within the range of from 0.1 wt.-% to 75 wt.-%, and
- carbohydrates other than D-allulose at a content within the range of from 1.0 wt.-% to 85 wt.-%,
in each case based on the total weight of the pet food composition on dry-matter basis.

2. The pet food composition according to claim 1, wherein the content of D-allulose is
- at least 0.050 wt.-%, preferably at least 0.1 wt.-%; and/or
- at most 5.0 wt.-%, preferably at most 2.5 wt.-%;
in each case based on the total weight of the pet food composition on dry-matter basis.

3. The pet food composition according to claim 1 or 2, which contains
- fat at a content within the range of from 8.0 wt.-% to 32 wt.-%, and/or
- protein at a content within the range of from 18 wt.-% to 50 wt.-%, and/or
- carbohydrates other than D-allulose at a content within the range of from 18 wt.-% to 74 wt.-%,
in each case based on the total weight of the pet food composition on dry-matter basis.

4. The pet food composition according to any of the preceding claims, which comprises an additive selected from the group consisting of preservatives, flavors, vitamins, mineral supplements, and combinations thereof.

5. The pet food composition according to claim 4, wherein the content of the additive is at most 45 wt.-%, based on the total weight of the pet food composition on dry-matter basis.

6. The pet food composition according to any of the preceding claims, which is
- a wet food; or
- a semi-moist food; or
- a dry food.

7. The pet food composition according to any of the preceding claims, which is
- a milk-product; and/or
- a cereal product; and/or
- a meat product; and/or
- a fish product.

8. The pet food composition according to any of the preceding claims, which comprises
- a prebiotic; and/or
- a food ingredient for which an EU Health Claim has been acknowledged; and/or
- a probiotic.

9. The pet food composition according to any of the preceding claims, wherein
(i) the total content of carbohydrates other than D-allulose is at most 5.0 wt.-%, preferably at most 2.5 wt.-%, more preferably at most 0.5 wt.-%, based on the total weight of the pet food composition on dry matter basis; and/or
(ii) the caloric value of the pet food is at most 6000 kcal/kg, preferably at most 5500 kcal/kg, more preferably at most 5000 kcal/kg, still more preferably at most 4500 kcal/kg, yet more preferably at most 4000 kcal/kg, even more preferably at most 3500 kcal/kg, most preferably at most 3000 kcal/kg, and in particular at most 2500 kcal/kg, in each case based on the total weight of the pet food composition on dry-matter basis; and/or
(iii) the pet food composition is selected from the group consisting of regular food, organic food, vegetarian food, vegan food, low carb food, low fat food, low protein food, low carb/low fat food, low carb/high fat food, low carb/low protein food, low carb/high protein food, low fat/low protein food, and high carb food.

10. The pet food composition according to any of the preceding claims, which comprises at most 5.0 wt.-% of L-allulose, relative to the total content of D-allulose contained in the pet food composition, preferably essentially no L-allulose.

11. The pet food composition according to any of the preceding claims, which comprises one or more mono- and/or disaccharides other than D-allulose, preferably wherein the content of D-allulose is greater than the total weight content of said one or more mono- and/or disaccharides other than D-allulose.

12. The pet food composition according to any of the preceding claims for use in the prevention or treatment of a condition in pets, wherein the condition is selected from the group consisting of impaired glucose tolerance, type 2 diabetes mellitus, hyperlipidemia, a metabolic syndrome, and obesity.

13. The pet food composition for use according to claim 12, wherein the pet is a dog or a cat.

14. Use of the pet food composition according to any of claims 1 to 11 as cat food or dog food.

15. Use of the pet food composition according to any of claims 1 to 11
- as pet food having reduced caloric content,
- as a dietetic pet food, and/or
- for improving the balance of the intestinal flora.
